(19) Europäisches Patentamt
European Patent Office
Office européen des brevets

(11) Publication number : 0 248 211 B1

# (12) EUROPEAN PATENT SPECIFICATION

(45) Date of publication of patent specification : 11.03.92 Bulletin 92/11

(51) Int. Cl.$^5$ : C12N 15/00, C07K 15/06, C12P 21/02, A61K 37/02

(21) Application number : 87106265.9

(22) Date of filing : 29.04.87

## (54) Novel protein and a method for production thereof.

(30) Priority : 30.04.86 JP 101531/86
04.09.86 JP 209091/86
05.09.86 JP 210429/86

(43) Date of publication of application : 09.12.87 Bulletin 87/50

(45) Publication of the grant of the patent : 11.03.92 Bulletin 92/11

(84) Designated Contracting States : BE CH DE FR GB IT LI LU NL SE

(56) References cited :
EP-A- 0 254 249
EP-A- 0 257 114
EP-A- 0 258 489
EP-A- 0 258 492
THE JOURNAL OF IMMUNOLOGY, vol. 129, no. 2, August 1982, pages 563-569, The American Association of Immunologists, US; F.M. MELEWICZ et al.: "Comparison of the Fc receptors for IgE on human lymphocytes and monocytes"
THE JOURNAL OF IMMUNOLOGY, vol. 132, no. 2, February 1984, pages 796-803, The American Association of Immunologists, US; D.H. CONRAD et al.: "The murine lymphocyte receptor for IgE. I. Isolation and characterization of the murine B cell Fc epsilon receptor and comparison with Fc epsilon receptors from rat and human"
CELL, vol. 47, no. 205, 2nd December 1986, pages 657-665, Cell Press; H. KIKUTANI et al.: "Molecular structure of human lymphocyte receptor for immunoglobulin E"
THE EMBO JOURNAL, vol. 6, no. 1, January 1987, pages 109-114, IRL Press Ltd, Oxford, GB; C. LÜDIN et al.: "Cloning and expression of the cDNA coding for a human lymphocyte IgE receptor"

(73) Proprietor : Yodoi, Junji
39, Kitashirakawa-Nishisenouchi-cho
Sakyo-ku Kyoto-City (JP)
Proprietor : KURARAY CO., LTD.
1621 Sakazu
Kurashiki-City Okayama Prefecture 710 (JP)
Proprietor : Honjo, Tasuku
Higashi-1-jo-agaru, Nishi-iru, Izumidono-cho
Yoshida, Sakyo-ku, Kyoto-shi 606 (JP)

(72) Inventor : Yodoi, Junji
39, Kitashirakawa-Nishisenouchi-cho
Sakyo-ku Kyoto-city (JP)
Inventor : Takami, Masaaki
1-1, Mizue
Kurashiki-city (JP)

(74) Representative : Vossius & Partner
Siebertstrasse 4 P.O. Box 86 07 67
W-8000 München 86 (DE)

EP 0 248 211 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The present invention relates to an IgE binding factor isolated from a culture medium of cells having an Fcε receptor and to a method for preparing the IgE binding factor.

Human IgE is an immunoglobulin which takes part in allergic reactions; some types of IgE are associated with asthma bronchia, allergic rhinitis, non-allergic Kimura's disease and other lung diseases as hyper IgE emia and some other types of IgE with autoimmune diseases, which have become a serious problem from a clinical aspect. An immunoglobulin comprises an Fab fragment which forms an antigen binding site and an Fc fragment for binding to the corresponding receptor (R). In mast cells the receptor FcεR is expressed; when IgE binds thereto, biologically active substances such as histamine, serotonin and SRS-A (Slow Reacting Substance of Anaphylaxis) are released and disease symptoms are induced by their biological activities. The isolation and characterization of the substances which participate in the regulation of IgE production, therefore is considered to possibly result in the provision of therapeutic and diagnostic agents for allergic diseases.

It has been reported by Ishizaka and Yodoi et al. that Fc∊R takes part in the regulation of IgE production. This was verified by the finding that IgE binding factor is produced from Fc∊R-positive T cells in experiments using rats [(J. Yodoi and K. Ishizaka: J. Immunol., 124, 1322, (1980)].

Journal of Immunology, Vol. 129, No. 2 (1982), pp. 563-569, discloses a human $Fc_ε$ receptor having a molecular weight of 47 000 which is immunoprecipitated with anti-$Fc_ε$ receptor serum. The document also discloses the co-immunoprecipitation of a protein having a molecular weight of 23 000.

Journal of Immunology, Vol. 132, No. 2 (1984), pp. 796-803, discloses a murine lymphocyte receptor for IgE and compares it with the corresponding human receptor with respect to molecular weight and isoelectric point.

It is also known that the substances which participate in the relation of IgE production comprise two active types (suppressive and enhancive) and these types are different from each other only in their sugar chain [K, Ishizaka, Lymphokines, 8, 41-80 (1983)].

On the other hand, the expression of FcεR has been investigated by T. Nagai et al., on Kimura's disease and atopic disease using the IgE-ORBC rosette method and H107 antibody. The results have revealed that the patients with hyper IgE emia show an FcεR positive rate as high as 10 to 20%, as compared to normal persons showing a rate not greater than 1% [T. Nagai et al.: Clin. Immunol. Immunopathol., 35, 261 (1985)].

Accordingly, the isolation and characterization of the genetic structure of an IgE binding factor could result in the provision of the factor as a drug; however,the IgE binding factor is produced only in trace amounts and can be separated and purified only with extreme difficulties. Therefore, an IgE binding factor has not yet been isolated and characterized.

The present inventors started from the assumption that an IgE binding factor would be a part of the FcεR, that the IgE binding factor would comprise a fragment homologous to the FcεR or that genetic information coding for the IgE binding factor would be present within the genetic information coding for the FcεR, because the IgE binding factor is produced from FcεR-positive cells. On the basis of this assumption it seemed desirable to purify a protein which is capable of reacting with an anti-Fcε receptor antibody from FcεR-positive cells on their culture supernatant, and to clarify its structure. Then, based on the knowledge of the clarified structure, DNA fragments could be synthesized and genetic information coding for the IgE binding factor or the FcεR protein could be isolated using the DNA fragments as a probe. Therefore, an object of the present invention is to provide a protein which is capable of reacting with an anti-FcεR antibody and to determine the structure of the protein.

The protein can be used for obtaining a gene coding for the IgE binding factor or of a gene coding for the entire FcεR using DNA fragments corresponding to a part of the amino acid sequence in such a protein as a probe and the determination of its structure. In this way the IgE binding factor or the FcεR could be obtained in large quantities by genetic engineering.

The object can be achieved by the present invention.

The subject matters of the invention therefore are:

(i) A protein in which a part of the amino acid sequence is represented by the polypeptide

Met-Glu-Leu-Gln-Val-Ser-Ser-Gly-Phe-Val

and which shows reactivity with an anti-Fcε receptor antibody.

(ii) A method for producing a protein in which a part of the amino acid sequence is represented by the polypeptide

Met-Glu-Leu-Gln-Val-Ser-Ser-Gly-Phe-Val

and which shows reactivity with an anti-Fcε receptor antibody which comprises culturing cells bearing a class-specific Fcε receptor on their surface and separating and harvesting the protein from the culture supernatant.

The protein can be used for obtaining the following:

(iii) A DNA having a nucleotide sequence represented by:

```
ATG GAG GAA GGT CAA TAT TCA GAG ATC GAG GAG CTT
CCC AGG AGG CGG TGT TGC AGG CGT GGG ACT CAG ATC   72
GTG CTG CTG GGG CTG GTG ACC GCC GCT CTG TGG GCT
GGG CTG CTG ACT CTG CTT CTC CTG TGG CAC TGG GAC  144
ACC ACA CAG AGT CTA AAA CAG CTG GAA GAG AGG GCT
GCC CGG AAC GTC TCT CAA GTT TCC AAG AAC TTG GAA  216
AGC CAC CAC GGT GAC CAG ATG GCG CAG AAA TCC CAG
TCC ACG CAG ATT TCA CAG GAA CTG GAG GAA CTT CGA  288
GCT GAA CAG CAG AGA TTG AAA TCT CAG GAC TTG GAG
CTG TCC TGG AAC CTG AAC GGG CTT CAA GCA GAT CTG  360
AGC AGC TTC AAG TCC CAG GAA TTG AAC GAG AGG AAC
GAA GCT TCA GAT TTG CTG GAA AGA CTC CGG GAG GAG  432
GTG ACA AAG CTA AGG ATG GAG TTG CAG GTG TCC AGC
GGC TTT GTG TGC AAC ACG TGC CCT GAA AAG TGG ATC  504
AAT TTC CAA CGG AAG TGC TAC TAC TTC GGC AAG GGC
ACC AAG CAG TGG GTC CAC GCC CGG TAT GCC TGT GAC  576
GAC ATG GAA GGG CAG CTG GTC AGC ATC CAC AGC CCG
GAG GAG CAG GAC TTC CTG ACC AAG CAT GCC AGC CAC  648
ACC GGC TCC TGG ATT GGC CTT CGG AAC TTG GAC CTG
AAG GGA GAG TTT ATC TGG GTG GAT GGG AGC CAT GTG  720
GAC TAC AGC AAC TGG GCT CCA GGG GAG CCC ACC AGC
CGG AGC CAG GGC GAG GAC TGC GTG ATG ATG CGG GGC  792
TCC GGT CGC TGG AAC GAC GCC TTC TGC GAC CGT AAG
CTG GGC GCC TGG GTG TGC GAC CGG CTG GCC ACA TGC  864
ACG CCG CCA GCC AGC GAA GGT TCC GCG GAG TCC ATG
GGA CCT GAT TCA AGA CCA GAC CCT GAC GGC CGC CTG  936
CCC ACC CCC TCT GCC CCT CTC CAC TCT
```

(iv) A protein obtained by the expression of a DNA having a nucleotide sequence coding for an amino acid sequence represented by:

Met Glu Glu Gly Gln Tyr Ser Glu Ile Glu Glu Leu
Pro Arg Arg Arg Cys Cys Arg Arg Gly Thr Gln Ile 24
Val Leu Leu Gly Leu Val Thr Ala Ala Leu Trp Ala
Gly Leu Leu Thr Leu Leu Leu Leu Trp His Trp Asp 48
Thr Thr Gln Ser Leu Lys Gln Leu Glu Glu Arg Ala
Ala Arg Asn Val Ser Gln Val Ser Lys Asn Leu Glu 72
Ser His His Gly Asp Gln Met Ala Gln Lys Ser Gln
Ser Thr Gln Ile Ser Gln Glu Leu Glu Glu Leu Arg 96
Ala Glu Gln Gln Arg Leu Lys Ser Gln Asp Leu Glu
Leu Ser Trp Asn Leu Asn Gly Leu Gln Ala Asp Leu 120
Ser Ser Phe Lys Ser Gln Glu Leu Asn Glu Arg Asn
Glu Ala Ser Asp Leu Leu Glu Arg Leu Arg Glu Glu 144
Val Thr Lys Leu Arg Met Glu Leu Gln Val Ser Ser
Gly Phe Val Cys Asn Thr Cys Pro Glu Lys Trp Ile 168
Asn Phe Gln Arg Lys Cys Tyr Tyr Phe Gly Lys Gly
Thr Lys Gln Trp Val His Ala Arg Tyr Ala Cys Asp 192
Asp Met Glu Gly Gln Leu Val Ser Ile His Ser Pro
Glu Glu Gln Asp Phe Leu Thr Lys His Ala Ser His 216
Thr Gly Ser Trp Ile Gly Leu Arg Asn Leu Asp Leu
Lys Gly Glu Phe Ile Trp Val Asp Gly Ser His Val 240
Asp Tyr Ser Asn Trp Ala Pro Gly Glu Pro Thr Ser
Arg Ser Gln Gly Glu Asp Cys Val Met Met Arg Gly 264
Ser Gly Arg Trp Asn Asp Ala Phe Cys Asp Arg Lys
Leu Gly Ala Trp Val Cys Asp Arg Leu Ala Thr Cys 288

Thr Pro Pro Ala Ser Glu Gly Ser Ala Glu Ser Met
Gly Pro Asp Ser Arg Pro Asp Pro Asp Gly Arg Leu 312
Pro Thr Pro Ser Ala Pro Leu His Ser

(v) A method for producing an IgE binding factor by the expression of a DNA comprising a nucleotide sequence fragment coding for the IgE binding factor which fragment is contained in the nucleotide sequence of the DNA shown in (iii).

(vi) A method for producing an IgE binding factor by the expression of a DNA comprising a nucleotide sequence fragment coding for the IgE binding factor which fragment is contained in the nucleotide sequence coding for the amino acid sequence show in (iv) .

(vii) A method for producing an IgE binding factor by the expression of the nucleotide sequence from numbers 448 to 963 in the nucleotide sequence of the DNA shown in (iii).

(viii) A method for producing an IgE binding factor by the expression of a DNA comprising the nucleotide sequence fragment coding for the amino acids from numbers 150 to 321 in the nucleotide sequence coding for the amino acid sequence shown in (iv).

The IgE binding factor obtained by the expression of the aforesaid DNA may be bound to a sugar chain (glycosylation).

Figure 1 Shows the result of a SDS-polyacrylamide gel electrophoresis of the protein obtained in Example 2. Figure 2 shows a western blot of the protein obtained through the purification with a H107 affinity column in Example 4 (2). Figure 3 shows the results of the determination of the amount of H107 antigen, the amount of IgE produced in an in vitro culture and the inhibition of rosette formation for each fraction of a gel-filtration of the protein obtained from the culture supernatant of RPMI 8866 cells in Example 5. Figure 4 shows the nucleotide sequence of the novel DNA obtained in Example 6 and the corresponding amino acid sequence. Figure 5 shows the structure of plasmid pKCR-ε R2A described in Example 7. Figure 6 shows the structure of plasmid pKCR-ε RSE described in Example 8.

In the present invention, the desired protein can be obtained by culturing cells harboring a class-specific FcεR on their surface and harvesting the protein from the culture supernatant. For the culture, there can be used human B cells, hybridoma thereof or human malignant B cells, human T cells, hybridoma thereof or human malignant T cells, human monocytes or human eosinophilic cells. Examples of human B cells include RPMI 8866 [Sarfati et al., Immunology, 53, 197 (1984)], and RPMI 1788 (ATCC catalogue No. CCL 156). Examples of human T cells include F18-19-19 [Microbiol. Immunol., 27 (10), 877-891 (1983)]. Of these cells, RPMI 8866 cells are well known. Further FcεR is induced by culturing B lymphocytes generally obtained from human peripheral blood in the presence of IgE and the cells obtained as such can also be used for the production of the aforesaid protein.

As medium used for culturing these cells, mention may be made of, for example, RPMI 1640 medium containing 10% FCS; using this medium, the cells described above are cultured at 30 to 38°C, preferably 36 to 37°C, at pH of 4 to 8, preferably pH of 7 to 8, in a $CO_2$ concentration of 3 to 10%, preferably 5 to 7%, for 1-3 days to reach a cell density of approximately $1x10^4$ to $1x\ 10^6$ counts/ml. These cells can also be cultured in a serum-free medium, if ,necessary and desired; from a viewpoint of purification of the protein, a serum-free medium is advantageous. The culture supernatant is subjected as it is or after concentration, to gel filtration, chromatography such as liquid chromatography,

affinity chromatography preferably using an anti-FcεR antibody, more preferably an anti-FcεR monoclonal antibody, for example, monoclonal antibody H107 as described in JP-A-255734/85. From this document it is known that H107 is a monoclonal antibody which specifically reacts with FcεR. When the isolation is carried out by affinity chromatography using a monoclonal antibody such as H107, it can be performed for example as follows: 2 liters of the culture supernatant of FcεR-containing cells, for example RPMI 8866, are passed through 0.2 ml of Sepharose 4B column to which H107 antibody has been bound. After washing with phosphate buffer, fractionation is performed with 0.5M NaCl and 0.1M acetic acid aqueous solution (pH 4.0). The desired fraction was concentrated followed by separation and purification using reverse phase high performance liquid chromatography.

In order to determine the amino acid sequence of the thus separated and purified protein, RPMI 8866 was cultured in a large scale and the supernatant was separated by passing through the H107 antibody-bound column as described above. After purification by reverse phase high performance liquid chromatography, analysis was performed by the Edman degradation method. The analysis can be manually performed but in order to analyze a trace amount of the protein with good accuracy, the vapor phase method is preferred. As a result of the analysis using, for example, a Gas Phase 470 Sequencer and 120 APTH Analyzer manufactured by Applied Biosystems Co., Ltd., a part of the amino acid sequence was determined to be Met-Glu-Leu-Gln-Val-Ser-Ser-Gly-Phe-Val.

The molecular weight of this protein can be determined by a band observed around 25 kD in SDS-polyacrylamide gel electrophoresis (hereafter simply referred to as SDS-PAGE). As a standard protein, a low molecular weight protein (for example from the Electrophoresis Calibration KIT manufactured by Pharmacia Co., Ltd.) can be employed. As will be described later, the molecular weight of the protein obtained in the examples has been verified to be 25,000 ± 2,000, taking the accuracy of the molecular weight measurement by SDS-PAGE into account.

Further an isoelectric point of the separated protein can be determined in the pH range of 3 to 10, using a protein of the Electric Focusing calibration KIT manufactured by Pharmacia Co., Ltd. as a PI marker, in accordance with the NEPHGE two dimensional electrophoresis described by B.B. Mishell and S.M. Shiigi, Selected Methods in Cellular Immunology, page 416 (1980), published by Freeman & Co. The isoelectric point of the protein thus separated is generally in a range of 5.4 to 6.0.

The properties of the obtained protein were examined using H107 antibody described above and it became clear that this protein inhibits the binding of H107 antibody to RPMI 8866. RPMI 8866 cells are cultured in RPMI 1640 medium supplemented with 1% FCS. The resulting culture supernatant is concentrated. The concentrate is passed through Sepharose 4B columns having bound thereto H107 antibody, human immunoglobulin and anti-Tac antibody, respectively and the liquid passed is mixed with $^{125}$I-labeled H107. Subsequently, RPMI 8866 cells are added to the mixture. After allowing to stand for a definite period of time, the cells are separated from the supernatant by centrifugation and, radioactivity of the cells and the supernatant is measured with a y counter. The results of the foregoing operation reveal that the effluent of the H107 antibody-bound Sepharose 4B columns does not inhibit the binding of $^{125}$I-labeled H107 antibody to RPMI 8866 but the culture supernatant passed through a column having bound human, immunoglobulin incapable of binding to this protein and anti-Tac antibody inhibits the binding of $^{125}$I-labeled H107 antibody to RPMI 8866. In addition, the culture supernatant of ATL-2 cells in which no FcεR has been expressed was concentrated and the concentrate was examined,by the sandwich RIA method of rabbit anti-RPMI 8866 cells antiserum and $^{125}$I-labeled H107 antibody; any antigen capable of reacting with H107 antibody was not detected but antigen was detected in the supernatant of RPMI 8866 cells.

Further the binding ability of the obtained protein to IgE was examined. To examine the binding ability, there are various methods; for example, the binding ability can be examined by inhibition of rosette formation. It has been made clear that rosette formation between IgE- coated erythrocytes (Eo′-IgE) obtained by binding human IgE to formalin-immobilized bovine erythrocytes according to the method of Ishizaka et al. [J. Immunol., 126, 1692 (1981)] and cells such as RPMI 8866 bearing FcεR capable of binding with IgE on the surface thereof is inhibited by this protein. This indicates that this protein has an ability of binding to IgE.

Furthermore, this protein was added to the culture medium of PWM-stimulated peripheral lymphocytes to examine the IgE production of the lymphocytes. As a result, the production of IgE was significantly increased as compared to the case where this protein was not added.

As described above, cells bearing a class-specific FcεR on their surface were cultured and a novel protein in which part of the amino acid sequence is represented by the polypeptide

Met-Glu-Leu-Gln-Val-Ser-Ser-Gly-Phe-Val

could be obtained from the culture supernatant. Such a protein reacts with an anti-Fcε receptor antibody and is thus useful as a protein for isolating genetic information coding for a human IgE binding factor and/or a human FcεR protein. As described above, a part of the amino acid sequence has been determined so that the corresponding gene can be synthesized based on the amino acid sequence and genetic information can be isolated using the same as a probe. It has also been made clear that the said protein is an IgE binding factor capable of binding to IgE and affecting IgE Production of B lymphocytes and that the said IgE binding factor is a part of FcεR.

As described above, an amino acid sequence can be optionally chosen which contains the amino acid sequence:

Met-Glu-Leu-Gln-Val-Ser-Ser-Gly-Phe-Val

which is a part of the aforesaid protein, to give a probe. As a length of the probe, 12 to 30 bases are generally used, considering the balance between a length which can avoid non-specific hybridization and a length that can be synthesized economically. Using such a probe, the desired gene, namely, a gene coding for an IgE binding factor can be obtained from a cDNA library obtained from the mRNA of the IgE binding factor-producing cells with reverse transcriptase. It is believed that the IgE binding factor is a part of FcεR since it reacts with the anti-FcεR monoclonal antibody H107. Therefore, to obtain the gene for the FcεR also means to obtain gene for the IgE binding factor. It is also possible to obtain genomic DNA, instead of cDNA, from a genomic DNA library using the aforesaid probe.

A method for isolating a gene coding for the entire length of the FcεR comprises several steps. Firstly, IgE binding factor-producing cells, for example, RPMI 8866 cells, are cultured as a source for supplying mRNA to collect approximately $10^8$ counts. The cells are homogenized together with guanidium thiocyanate and overlaid onto a CsCl solution followed by ultracentrifugation, whereby the whole mRNA is separated (Chirgwin et al., Biochemistry, 18, 5294-5299 (1979)]. Then, the mRNA is purified by oligo (dT) cellulose column chromatography to give poly (A) RNA. This mRNA is treated according to the Okayama-Berg method [Okayama and Berg, Molecular and Cellular Biology, 2, 161-170 (1982)] to give a cDNA library. Namely, a vector primer and an oligo (dG) tail linker are prepared. Reverse transcriptase is employed in the presence of this vector primer and the mRNA described above to synthesize the cDNA. Then the cDNA is digested with the restriction endonuclease Hind III followed by cyclization using the linker described above. Subsequently, the mRNA fragment is replaced with DNA to give a cDNA fragment-containing plasmid. Then, the plasmid is transformed into Escherichia coli in a conventional manner to harvest ampicillin-resistant cells; the cells are made a cDNA library. On the other hand, as an example for a nucleotide corresponding to the amino acid sequence that is a part of the IgE binding factor, a mixture of 32 oligonucleotides 14-mer corresponding to Met----Val in the amino acid sequence described above is synthesized; using this probe, the aforesaid cDNA library is subjected to screening by in situ hybridization in order to select a clone hybridizing with the probe. The thus obtained clones have a length of 1.1 kb and 1.7 kb. Of these, the nucleotide sequence of the cDNA having a length of 1.7 kb was determined according to the dideoxy method by Sanger et al. (Proc. Natl. Acad. Sci. USA, 74, 5463 (1977)]. By analyzing the sequence, it has been clarified that the obtained cDNA clone codes for the entire length of FcεR. It has been confirmed that the amino acid sequence deduced from this cDNA included the amino acid sequence which was a part of the protein separated from the culture supernatant described above. It has thus made been clear that the IgE binding factor in the culture supernatant is a part of the FcεR on the cell surface.

The transformation into a microorganism, for example, Escherichia coli, for purpose of expressing the FcεR-cDNA thus obtained, can be achieved by inserting the FcεR-cDNA into an appropriate vector, for example, pKK 223-3 (manufactured by Pharmacia Co., Ltd.) and introducing the vector into Escherichia coli. If, for example, the plasmid pKCRH2 (Mishima et al., Nature, 307, 604-608 (1984)) is used as a vector the FcεR-cDNA can be expressed in animal cells. It was further confirmed by the following experiment that this cDNA coded for FcεR. Using the plasmid pKCrH2, Cos-7 cells were selected as animal cells and the plasmid was introduced by the $Ca^{++}$ phosphate method. After incubation of the cells for 2 to 3 days,the expression of the FcεR was determined by two methods. After the incubation the cells were reacted with H107 antibody and then reacted with FITC-labeled anti-mouse IgE antibody. When fluorescence stained cells were assayed by fluorescence microscopy, approximately 5% of the cells were positive. Further in order to determine the binding ability to IgE, the cells were reacted with human IgE and then reacted with FITC-labeled anti-human IgE antibody. When the cells were assayed by fluorescence microscopy, approximately 4% of the cells were positive. On the oder hand, in COS-7 cells in which the FcεR gene is inserted in reverse direction, no fluorescence was observed. From the foregoing, it has become clear that this cDNA is a gene coding for FcεR and, the gene is expressed and FcεR is produced in the transformed cells. This has also been confirmed by flow cytometry as will be later described in Example 8. It has also become clear that an IgE binding factor is produced in the culture supernatant of COS-7 cells transfected with FcεR-cDNA by measuring the culture supernatant according to the sandwich ELISA method. It has further become clear that addition of the obtained IgE binding factor into the culture medium of IgE-producing myeloma cells U 266 enhances the production of IgE. Thus, there can be obtained a DNA coding for FcεR and an IgE binding factor, and the protein is expressed by using the DNA coding for the FcεR and the IgE binding factor. By introducing the aforesaid gene into bacteria, animal cells or plant cells, it has also become possible to obtain the IgE binding factor and the FcεR in large quantities. The products obtained can be used for the treatment of allergy by eliminating an excess of IgE from the blood of the allergy patient having a higher IgE concentration in blood than the normal level. utilizing their binding ability to IgE; in addition thereto, also utilizing their binding ability to IgE, the products can be used for diagnosis such as detection or quantitative determination of IgE in blood or specimen.

Examples

Example 1

RPMI 8866 cells were cultured at a temperature of 37°C and a $CO_2$ concentration of 7% in RPMI 1640 medium (RPMI 1640 medium manufactured by GIBCO Co., Ltd., supplemented with 100 μg/ml streptomycin sulfate and 100 u/ml of penicillin G potassium) containing 10% FCS (fetal calf serum) to reach a cell density of 5 x $10^5$ cells/ml. 2 liters of the resulting culture medium were centrifuged to separate the culture supernatant from the cells. The obtained culture supernatant was passed through a column with 0.2 ml of Sepharose 4B carrying H107 antibody. The H107 antibody-bound column was prepared as follows. 5 mg of H107 was reacted with 1 ml of swollen gel, 0.1M $NaHCO_3$ and 0.5M NaCl aqueous solution (pH 8.0) at 4°C overnight according to the data sheet of Tresyl-Sepharose 4B manufactured by Pharmacia Co., Ltd. The unreacted active group was blocked by reacting at 4°C overnight in 0.1M Tris-HCl buffer solution (pH 8.0). Thereafter, the system was washed with 0.1M acetic acid having pH of 4.0 and 0.5M NaCl and with 0.1M Tris-HCl buffer solution having pH of 8.0 and 0.5M NaCl, alternatively, 3 times and lastly washed with phosphate buffered saline (PBS). Using the obtained H107 antibody-bound column, the culture supernatant was passed through the column. After the column was washed with 10 ml of phosphate buffer (pH 7.5) containing 0.5M NaCl, the column was eluted with 0.5M NaCl and 0.1M acetic acid aqueous solution (pH 4.0). After completion of the elution, the eluate was immediately neutralized with 2M Tris base and concentrated using Centricon 10 manufactured by Amicon Co., Ltd. At the same time, the buffer was replaced with water and separation was performed by reverse phase high performance liquid chromatography [Synchropak RD-PC 18 column, 0.1% trifluoroacetic acid (TFA), water-acetonitrile linear gradient]. According to Selected Method in Cellular Immunology [Mishell and Shiigi, page 434 (1980), published by Freeman & Co.], the purified protein was put on 15% polyacrylamide gel followed by electrophoresis at a current of 25 mA for 3 hours using as a buffer 25 mM of Tris, 190 mM of glycine and 0.1% of SDS. As standard protein, the aforesaid Electrophoresis calibration KIT manufactured by Pharmacia Co., Ltd. was used. As the result, 100 ng of protein showing 2 bands close to each other at about 25 kD was obtained (which was presumed from the density of the bands by silver staining of SDS-PAGE). Taking the ability of SDS-PAGE for separating protein into account, the molecular weight of the protein found at about 25 kD is considered to be 25,000 ± 2,000.

Example 2

RPMI 8866 cells were cultured in serum-free medium (manufactured by Hana Co., Ltd. ) to reach a cell density of 5 x $10^5$ cells/ml and the culture medium was centrifuged to give the culture supernatant. Using CH2PR-HIP 10-20 hollow fiber system manufactured by Amicon Co., Ltd., 10 liters of the culture supernatant was concentrated to 400 ml and the concentrate was passed through a column with 0. 4 ml of Sepharose 4B carrying H107 antibody. Thereafter, the system was washed with 20 ml of phosphate buffer (pH 7.5) containing 0.5M NaCl and 0.1% of NP 40 and then eluted with 0.5M NaCl, 0.1% NP 40, and 0.1M acetic acid aqueous solution(pH 4.0). The eluate was separated and purified by reverse phase high performance liquid chromatography in a manner similar to Example 1 and then subjected to SDS-PAGE under the same conditions as in Example 1. As the result, 3 μg of protein showing 2 bands close to each other at about 25 kD was obtained. SDS-PAGE is shown in Figure 1. In Figure 1, lane A shows the protein obtained by purification with H107 affinity column and lane B shows a molecular weight marker.

Example 3

The protein separated and purified by reverse phase high performance liquid chromatography in Example 2 was subjected to amino acid sequence analysis using Gas Phase 470A Sequencer and 120A APTH Analyzer manufactured by Applied Biosystems Co., Ltd. As the result, the amino acid sequence of the two bands close to each other found by SDS-PAGE at about 25 kD both contained the partial sequence Met-Glu-Leu-Gln-Val-Ser-Ser-Gly-Phe-Val. An isoelectric point of these proteins was determined in a pH range of 3 to 10 using protein in Electric Focusing Calibration KIT manufactured by Pharmacia Co., Ltd. as a PI marker by the NEPHGE two dimensional electrophoresis described above. As the result, a PI of 5.50 in the former protein and of 5.85 in the latter protein was found.

Example 4

It was verified by the following facts that the proteins obtained in Examples 1 and 2 were antigens specific to H107 antibody which recognized FcεR and antigens intrinsic to RPMI 8866 cells which expressed FcεR in large quantities.

(1) The culture supernatant obtained by culturing RPMI 8866 cells in 1% FCS-containing RPMI 1640 medium to reach a cell density of 5 x $10^5$ cells/ml was concentrated to 75 times using CH2PR-HIP 10-20 hollow fiber system manufactured by Amicon Co., Ltd. The concentrate was passed through Sepharose 4B columns (0.2 ml of each column was used based on 50 ml of the concentrated culture supernatant) having bound thereto 3 kinds of antigens, i.e., H107, human immunoglobulin and anti-Tac antibody, respectively. A relative amount of H107 antigen present in the culture supernatant and in each effluent was calculated in such a ratio that inhibited the binding of H107 antibody to the RPMI 8866 cells. Namely, 50 μl of $^{125}$I-labeled H107 antibody (5000 cpm) and 20 μl each of the concentrated culture supernatant, the effluent and each dilution thereof were mixed and the mixture was allowed to stand for 30 minutes under ice cooling. Then 50 μl of a solution containing 1 x $10^6$ counts of RPMI 8866 cells was added thereto. The mixture was gently mixed and allowed to stand for 30 minutes under ice cooling. The mixture was overlaid on a mixture of 20% olive oil and 80% dibutyl phthalate followed by centrifugation. With respect to the cell pellet and the supernatant, cpm was determined by a γ counter and a binding rate of the $^{125}$I-labeled H107 antibody was calculated. As the result, the count of the cell pellet portion in the liquid passed through the H107 antibody-bound column was 1970 cpm and the count of the supernatant was 750 cpm. The count of the cell pellet portion in the liquid passed through the human immunoglobulin-bound column was 570 cpm. The count of the cell pellet portion in the liquid passed through the anti-Tac antibody-bound column was 700 cpm. Therefore, H107 antigen present in the culture supernatant of the RPMI 8866 cells was not adsorbed on human immunoglobulin and anti-Tac antibody but absorbed on H107 antibody.

(2) The fraction obtained from the culture supernatant of RPMI 8866 cells by affinity chromatography using H107 antibody was subjected to western blot to detect H107 antigen. For this purpose the crude fraction eluted from the H107 antibody-bound Sepharose 4B column in Example 2 was subjected to 15% SDS-PAGE under non-reducing condition followed by electro-blot on a nitrocellulose membrane. After the nitrocellulose membrane was blocked by bovine serum albumin (BSA), the membrane was reacted with H107 antibody and then reacted with goat anti-mouse IgG antibody-HRP. Thereafter, a color was formed by hydrogen peroxide and diaminobenzidine. As the result, 2 bands close to each other were detected at about 25 kD. The western blot is shown in Figure 2. In Figure 2, lane A shows the 2 bands detected as described above, lane B shows an example in which no reaction was caused with H107 antibody and lane C shows an example in which mouse IgG was used under reducing conditions in place of this protein.

(3) The amounts of H107 antigen in the culture supernatants were compared between RPMI 8866 cells and ATL-2 cells (expression of FcεR was not noted) (ATL-2 means human leukemia cell). That is, RPMI 8866 and ATL-2 were cultured in HB101 serum-free medium (manufactured by Hana Co., Ltd.) and concentrated to 58 times and 66 times, respectively. Then the amounts of H107 antigen were determined by the sandwich RIA method using rabbit anti-RPMI 8866 cell antibody and $^{125}$I-labeled H107 antibody, respectively, and compared with each other. As the result, no H107 antigen was detected in the culture supernatant of ATL-2. On the other hand, H107 antigen was significantly present in the culture supernatant of RPMI 8866.

Example 5

FCS (fetal calf serum) was added to the affinity purified 25 kD proteins up to a concentration of 1%. The mixture was fractionated by 0.5 ml each using Sepharose for a gel filtration column. With respect to each fraction, the amount of H107 antigen, activity for inhibiting rosette formation of ox red blood cell-bound IgE (Eo′-IgE) to RPMI 8866 cells and influence on IgE production of PWM-stimulated human peripheral blood were examined.

An assay for the amount of H107 antigen was carried out as follows. A 96 well plate for ELISA was used; 2 μg/ml solution of rabbit anti-RPMI 8866 cells antibody (IgG fraction) purified by Protein A Sepharose in coupling buffer (0.1M $NaHCO_3$, adjusted pH to 9.6 with NaOH) was charged in each well by 200 μl each followed by incubation at 4°C for 4 hours. After washing 7 times with washing buffer (PBS, pH 7.5, Tween 20, 0.05%), 200 μl of 3% BSA (bovine serum albumin)/washing buffer solution was added thereto and followed by incubation at 4°C for 4 hours. Then, after washing 7 times with washing buffer, a specimen diluted to each dilution was added by 100 μl each followed by incubation at 4°C for 4 hours. Thereafter, the system was washed with washing buffer 7 times. 100 μl of alkali phosphatasebound H107 antibody (1.3 μg/ml, manufactured by Nichiray Co.,Ltd.) was added , followed by incubation at 4 °C for 4 hours. After washing with washing buffer 7 times, 100 μl each of a substrate solution [phosphate substrate tablet (Sigma Chem. Co., USA) was dissolved in 5 ml of develop buffer (97 ml of diethanolamine, 100 mg of $MgCl_2.6H_2O$ and 200 mg of $NaN_3$ were made 1 liter with distilled water)] was added to form a color at room temperature. The color density was determined at 2 wavelengths, OD 405-660 nm by an ELISA Reader. The fraction in which H107 antigen was detected was present at a molecular weight marker of about 20 kD (cf. Figure 3).

The inhibition of rosette formation was carried out as follows. Formalin-immobilized bovine blood cells (10% PBS solution) prepared according to the method of Ishizaka et al. [J. Immunol., 126, 1692 (1981)) were dispersed in acetate buffer (0.1M AcOH, pH 5.0). The dispersion, 250 μl, was mixed with 250 μl of human IgE (1 mg/ml) and 500 μl of acetate buffer followed by incubation at room temperature for 2 hours. After washing with PBS 3 times, the system was dispersed in 1 ml of Dulbecco-modified Eagle's medium (Eo′-IgE). To 25 μl of the Eo′-IgE dispersion was added 25 μl of each fraction followed by incubation at room temperature for 30 minutes. To the resulting mixture was added 25 μl of a solution previously prepared so as to be 5 x $10^6$/ml of RPMI 8866 cells in 2% FCS-containing RPMI 1640 medium followed by incubation at 37°C for 10 minutes. After centrifuging at 2500 rpm for 2 minutes using a desk type micro centrifug machine, the system was allowed to stand for 2 hours in ice. Cells having bound thereto at least 3 Eo′-IgEs were microscopically counted as rosette forming cells; when a rosette formation rate was made 100 in case that no specimen was added, the inhibition ratio of rosette formation of each fraction was calculated. The fraction which inhibited rosette formation was present at about 20 kD with the molecular weight marker (cf. Figure 3).

In order to examine the influence of PWM-stimulated peripheral blood on IgE production, the following experiment was carried out. Human peripheral blood, 50 ml, was diluted with 50 ml of PBS and the dilution was added to 50 ml of Ficoll paque. The mixture was centrifuged at 1500 rpm for 30 minutes to separate the lymphocyte fraction. The lymphocytes were washed with PBS once and further with RPMI 1640. The lymphocytes were suspended in 10% FCS-containing RPMI 1640 in a cell concentration of 1 x $10^6$/ml and 200 μl each of the suspension was separately charged in a 96 well microplate. To the culture medium were added 10 μg/ml of PWM and 0.01% cowan I. In addition, 5 μl each of each fraction described above was added to the mixture. After incubating at 37°C for 6 days in a $CO_2$ incubator, IgE in the culture medium was assayed by the ELISA method. That is, 100 μl each of 250 ng/ml (prepared with 0.1M $NaHCO_3$, pH 9.6, controlled with NaOH) of rabbit anti-human IgE (manufactured by DAKO Co., Ltd.) was charged in a 96 well plate for ELISA followed by incubation at 4°C for 4 hours and then washing with washing buffer 7 times. Then, 200 μl each of washing buffer containing 3% BSA was added thereto. After incubating at 4°C for 4 hours, the system was washed with washing buffer 7 times. The culture supernatant previously prepared was diluted with 0.1% BSA-containing washing buffer to 3-fold and 100 μl each of the dilution was added followed by incubation at 4°C for 4 hours. After washing with washing buffer 7 times, a 1000-fold dilution of alkali phosphatase-bound goat anti-human IgE (manufactured by Tago Co., Ltd.) diluted with 0.1% BSA-containing washing buffer was added by 100 μl each followed by incubation at 4°C for 4 hours. After washing with washing buffer 7 times, the substrate solution (supra) was added by 100 μl each to form a color at room temperature. Measurement was performed at 2 wavelengths of OD 405-660 using ELISA Reader and the data was compared with a calibration curve previously determined to quantitatively determine the IgE produced. The activity for enhancing IgE production was noted at about 20 kD (cf. Figure 3).

Example 6

(i) RPMI 8866 cells, 1 x $10^8$, were added to 10 ml of 4M guanidium thiocyanate solution (pH 7.0) containing 25 mM sodium citrate, 0.5% N-lauroyl sarcosine and 100 mM 2-mercaptoethanol, which was homogenized. The obtained homogenate was overlaid on 5.7 M CsCl according to the method of Chirgwin et al. [Biochemistry, 18, 5294-5299 (1979)] followed by ultracentrifugation, whereby the whole RNAs were separated. Then, the RNAs were subjected to oligo (dT) cellulose column chromatography and eluted in 1 ml of a solution containing 10 mM Tris-HCl (pH 7.5), 1 mM EDTA and 0.05% SDS, which was made a mRNA raw material.

(ii) On the other hand, vector primer and oligo (dG) tail linker were obtained using hybrid plasmid of pBR322 and SV40 according to the method of Okayama and Berg [Molecular and Cellular Biology, 2, 161-170 (1982)].

Namely, 400 μg of hybrid plasmid of pBR322 and SV40 (map unit 0.71 - 0.86),was digested with restriction enzyme Kpn I at 37°C for 4 hours in bovine serum albumin-containing buffer.

Then, DNA was recovered by precipitation with ethanol and dissolved in dTTP-containing buffer. Terminal deoxynucleotidyl transferase was added to the solution. The mixture was reacted at 37°C for 30 minutes to add approximately 60 dT tails at the site digested with restriction enzyme Kpn I. Thereafter, DNA was recovered by precipitation with ethanol. Then, this DNA was digested (37°C, 5 hours) with restriction enzyme Hpa I in bovine serum albumin-containing buffer. The larger DNA fragment was purified by agarose gel electrophoresis and recovered by the glass powder method [Vogelstein et al., Proc. Natl. Acad. Sci. USA, 76, 615-619 (1979)]. Then, this DNA was passed through oligo (dA) cellulose column at 0°C. After eluting with water, recovery was effected with ethanol to give vector primer bearing oligo (dT) tail.

On the other hand, 100 μg of hybrid plasmid of pBR322 and SV40 (map unit 0.19 - 0.32) was digested with restriction enzyme Pst I (37°C for 1 hour and a half) in bovine serum albumin-containing buffer. Then, DNA was recovered and dissolved in dGTP-containing buffer. Terminal deoxynucleotidyl transferase was added to the solution. The mixture was reacted at 37°C for 20 minutes to add approximately 10 to 15 dG tails thereto. The recovered DNA was digested (37°C, 1 hours) with restriction enzyme Hind III in bovine serum albumin-containing buffer and then subjected to agarose gel(1.8%) electrophorasis to extract and recover small oligo (dG) tail linker DNA.

(iii) cDNA library was obtained according to the method of Okayama and Berg [Molecular and Cellular Biology, 2, 161-170 (1982 ) ].

Namely, 5 μg of mRNA obtained in (i) described above and 2 μg of vector primer obtained in (ii) described above were added to an aqueous solution containing Tris-HCl (pH 8.3), $HgCl_2$, KCl, dithiothreitol, dATP, dTTP, dGTP and α - [32P] dCTP. The mixture was reacted at 37°C for 20 minutes in the presence of reverse transcriptase to synthesize plasmid cDNA : mRNA, which was followed by precipitation with ethanol to recover in a pellet state. The pellet was dissolved in a buffer containing $CoCl_2$, dithiothreitol, poly (A),α - [32P] dCTP and terminal deoxynucleotidyl transferase followed by reacting at 37°C for 10 minutes thereby to add 10 to 15 residues of dCMP.

Then, the recovered pellet containing oligo (dC)-tailed plasmid cDNA : mRNA was dissolved in bovine serum albumin-containing buffer and digested with restriction enzyme Hind III at 37°C for an hour. By precipitation with ethanol, HIND III-digested oligo (dC)-tailed cDNA : mRNA plasmid was recovered.

The plasmid was dissolved in oligo (dG)-tailed linker DNA-containing buffer obtained in (ii) described above followed by incubation at 65°C for 2 minutes and maintaining at 42°C for further 30 minutes. The system was then cooled to 0°C. Subsequently, E. coli DNA ligase was added thereto followed by incubation overnight in the presence of β-NAD (nicotine adenine dinucleotide). Thereafter, dATP, dTTP, dGTP, dCTP, β-NAD, E. coli DNA ligase, E. coli DNA polymerase and RNase H were added thereto. After incubation at 12°C for an hour and then at room temperature for an hour, the mixture was cooled to discontinue the reaction, whereby the desired cDNA fragment-containing plasmid was obtained. Then, E. coli HB101 [ATCC 33694] was transformed using this plasmid in a conventional manner to give $10^5$ colonies resistant to ampicillin.

On the other hand, Met-Glu-Leu-Gln-Val was chosen from the amino acid sequence:

Met-Glu-Leu-Gln-Val-Ser-Ser-Gly-Phe-Val

of protein separated from the culture supernatant and having reactivity with anti-FcεR antibody and a mixture of 32 kinds of 14-mers of the corresponding DNA sequence:

```
5'-ATG-GAA-CTA-CAA-GT-3'
       G T C   G
           G
           T
```

was synthesized by the solid phase method. The 5′-end was labelled with 50 picomols of the synthesized oligo DNA and 150 μCi (SP 3000 Ci/mmol) of $\gamma^{32}$ P ATP using $T_4$ polynucleotide kinase in a conventional manner. The activity obtained was 1.1 x $10^7$ cpm/50 pmol. Colony hybridization was conducted according to the method of Grunstein et al. [Proc. Natl. Acad. Sci., 72, 3961 ′1975)] and as the result, 2 clones which hybridized with the probe were selected; clones having lengths of 1.1 kb and 1.7 kb were obtained. From each clone, cDNA was extracted and a restriction map was prepared. cDNA of 1.1 kb was contained in 1.7 kb.

(iv) The nucleotide sequence of the cloned cDNA was determined by the dideoxy method [F. Sanger et al., Proc. Natl. Acad. Sci., USA, 74, 5463 (1977)]. The plasmid containing the cloned cDNA was digested with a suitable restriction enzyme and inserted into M13 mp7 (manufactured by Pharmacia Co., Ltd.) phage-reproduced DNA with $T_4$ DNA ligase. Then, the system was transfected into E. coli JM 103 [ATCC 39403]. Colorless plague was selected and proliferated to give template DNA. This template DNA, 5 μl (50 μg/ml), 1 μl (2.5 μg/ml) of single stranded 15 base primer, 1 μl (50 mM) of NaCl, 1 μl (10 mM) of $MgCl_2$, 1 μl (10 mM, pH 7.5) of Tris-HCl and 1 μl (1 mM) of dithiothreitol were charged in 10 μl of a capillary (Clay Adams) to anneal the primer. Then, the complimentary strand was elongated using DNA polymerase I large fragment. In this case, $\alpha^{32}$P dATP, dCTP, dGTP, dTTP and one selected from ddATP, ddCTP, ddGTP and ddTTP were added to make 4 kinds of systems in total.

The thus obtained complimentary strands having various lengths were separated by electrophoresis and subjected to autoradiography, whereby the pattern was analyzed to determine the nucleotide sequence as shown in Figure. The a nalysis of the nucleotide sequence of the thus obtained FcεR cDNA revealed that the initiation codon ATF was present at base No. 214 to initiate a region coding for the protein and a sequence corresponding to the probe used was present starting from base No. 661 (cf. the underlined part in Figure 4). Further a sequence identical with the N-terminal amino acid sequence of the aforesaid protein which is capable of reacting with anti-FcεR antibody was confirmed, from which it was understood that this protein was the IgE binding factor. In addition, the protein was considered to be a glycoprotein because a site capable of binding a sugar to form an N-bound type glycoprotein was present at base No. 400 and a large number of sites capable of binding sugars to form an O-bound type glycoprotein were present and also because the molecular weight of FcεR was 38 kD presumed from its length corresponding to base No. 214 to 1176 (approximately 43000 as previously presumed by Loren, H. et al). Likewise, this protein comprises the IgE binding factor In addition, the protein was considered to be a glycoprotein because in the sequence from base No. 661 to 1176 corresponding to the IgE binding factor, a large number of sites capable of binding sugars to form an O-bound type glycoprotein were present and also because the molecular weight of the IgE binding factor presumed from the sequence was 20 kD, which approximated to the value of 25000 previously determined.

In Figure 4, the symbols represent the following meanings:

| | |
|---|---|
| (Nucleotides) A | Deoxyadenylic acid |
| | G Deoxyguanylic acid |
| | T Thymidylic acid |
| | C Deoxycytidylic acid |
| (Amino acids) A | Alanine |
| | C Cysteine |
| | D Asparaginic acid |
| | E Glutamic acid |
| | F Phenylalanine |
| | G Glycine |
| | H Histidine |
| | I Isoleucine |
| | K Lysine |
| | L Leucine |
| | M Methionine |
| | N Asparagine |
| | P Proline |
| | Q Glutamine |
| | R Arginine |
| | S Serine |
| | T Threonine |
| | V Valine |
| | W Tryptophane |
| | Y Tyrosine |

Example 7

The hybrid plasmid bearing the cloned cDNA of 1.7 kb was cleaved with EcoR V to make it linear. Then, partial cleavage was effected with Pvu II to give a fragment of approximately 1.9 kb. This fragment was then treated with DNA polymerase I and Klenow fragment. The plasmid pKCRH2 was cleaved with Hind III to make it linear and then treated with DNA polymerase I and Klenow fragment and further treated with bacterial alkali phosphatase. These vectors and the 1.9 kb fragment were ligated with each other to give the expression vector pKCR-εR-2A (cf. Figure 5). At the same time, vector pKCR-εR-2B in which the 1.9 kb fragment had been inserted in the opposite direction was obtained.

On a culture dish having a diameter of 9 cm charged with 10 ml of Dulbecco-modified Eagle's medium containing 10% FCS, 3 x $10^5$ counts of COS-7 cells [ATCC CRL 1651] (Cell, 23, 175-182 (1981)] subcultured in Dulbecco-modified Eagle's medium containing 10% FCS were inoculated and incubated at 37°C for 20 hours. The medium was replaced and 4 hours after 1 ml of transfection solution (20 μg DNA/ml, $Ca^{++}$, HEPES sodium phosphate) [Wegler et al., Proc. Natl. Acad. Sci., USA, 76, 1373 (1979)] was supplemented. After incubation for 12 hours, the COS-7 cells were treated with PBS containing 2.5% glycerol at room temperature for 1 minutes and then washed twice with PBS.

Further 10 ml of 10% FCS-containing Dulbecco-modified Eagle's medium was supplemented followed by incubation for 40 hours. The cells were collected and the expression of FcεR was assayed with a fluorescence microscope after staining with antibody labeled with FITC (fluorescent dye). The staining was carried out as follows.

5 x $10^5$ counts of COS-7 cells were incubated at 4°C for 30 minutes with 10 μl of H107 antibody (10 μg/ml). The COS-7 cells were washed with 1% FCS-containing Hanks' solution. Then incubation was performed at 4°C for 30 minutes together with 20 μl of a 80-fold dilution of FITC-labeled goat anti-mouse IgG (manufactured by Tago Co., Ltd.). After washing with 1% FCS-containing Hanks' solution, the assay was performed with a fluorescence microscope. Binding with IgE was performed also in a similar manner. For this purpose, 5 x $10^5$ counts of COS-7 cells were incubated at 4°C for 30 minutes together with 20 μl of human IgE (100 μg/ml). The COS-7 cells were washed with 1% FCS-containing Hanks' solution. Then incubation was performed at 4°C for 30 minutes with 10 μl of FITC-labeled goat anti-human IgE antibody (manufactured by Tago Co., Ltd.). After washing with 1% FCS-containing Hank's solution, the assay was performed with a fluorescence microscope. As a positive control, expression of pKCR-Tac 2A [Nikaido et al., Nature, 311, 631 (1984)] was assayed in a similar manner using 20 μl of FITC-labeled mouse anti-Tac antibody. The results are shown in Table 1.

FcεR was expressed by COS-7 cells transfected with FcεR-cDNA, which was detected with anti-FcεR antibody H107, and at the same time, its binding ability to IgE was also confirmed.

Table 1

Expression of FcεR-cDNA in COS-7 Cells

| DNA | Antibody (1) | Antibody (2) | Positive Cell % |
|---|---|---|---|
| 1a pKCR-εR-2A | H107 | αm.IgG-FITC | 23/491, 4.7 |
| b | αTac | αm.IgG-FITC | 0/311, 0 |
| c | IgE | αh.IgE-FITC | 11/294, 3.7 |
| d | αm.IgG-FITC | - | 0/213, 0 |
| e | αh.IgE-FITC | - | 0/310, 0 |
| 2a pKCR-εR-2B | H107 | αm.IgG-FITC | 0/351, 0 |
| b | IgE | αh.IgE-FITC | 0/298, 0 |
| 3 pKCR-Tac2A | αTac-FITC | - | 29/378, 7.7 |

Example 8

The hybrid plasmid bearing the cloned cDNA of 1.7 kb was cleaved with Sal I to make it linear. Then, partial cleavage was effected with Pvu II to give a fragment of approximately 1.6 kb. Using this fragment the expression vector pKCR-εR-SE was obtained in a manner similar to Example 7 (cf. Figure 6).

COS-7 cells were transfected with pKCR-εR-SE in a manner similar to Example 7 followed by incubation for 40 hours. After incubating with H107 antibody and then with FITC-labeled goat anti-mouse IgG, FcεR expressed on the transfected COS-7 cells was detected by flow cytometry (Ortho Spectrum III). The results are shown in Table 2.

Also from the results, it is understood that FcεR-cDNA was expressed in the COS-7 cells and detected by anti-FcεR antibody H107 and at the same time, the binding ability to IgE was also confirmed.

Table 2

Expression of FcεR-cDNA in COS-7 Cells

| DNA | Antibody (1) | Antibody (2) | Positive Cell % |
|---|---|---|---|
| 1a pKCR-εR-SE | H107 | αm.IgG-FITC | 16 |
| b | αTac | αm.IgG-FITC | 0 |
| c | IgE | αh.IgE-FITC | 10 |
| d | αm.IgG-FITC | - | 0 |
| e | αh.IgE-FITC | - | 0 |
| 2a pKCR-εR-2B | H107 | αm.IgG-FITC | 0 |
| b | IgE | αh.IgE-FITC | 0 |
| 3 pKCR-Tac2A | αTac-FITC | - | 25 |

Next, the IgE binding factor in the culture supernatant of the COS-7 cells transfected with Fcε R-cDNA was quantitatively determined by the sandwich ELISA method using two different anti-FcεR antibody H107 and Mab 176 (JP-A-289100/86).
It was 170 pg/ml, which was a considerably low yield as compared to RPMI 8866 cells(10 ng/ml), but its expression was confirmed. No expression was confirmed in the COS-7 cells transfected with pKCRH2. Further it was examined by adding the culture supernatant and the IgE binding factor purified by affinity chromatography to the culture system of myeloma cells U266 as to whether or not the expressed IgE binding factor had a function of regulating the IgE Production. In any case, an increase in the IgE production was noted. The results are shown in Table 3.

Table 3

IgE Response of IgE binding factor Expressed in COS-7 Cells Transfected with FcεR-cDNA

| Samples | Concentration of IgE binding factor | Produced IgE |
|---|---|---|
| Conditioned medium of COS-7 cells transfected with: | | |
| pKCRεRSE | 33 pg/ml | 1712±182 ng/ml |
| pKCRH2 | 0 | 906±116 |
| Affinity purified IgE binding factor expressed in COS-7 cells transfected with: | | |
| pKCRεRSE | 20 | 1840±284 |
| pKCRH2 | 0 | 890±120 |
| Medium | 0 | 1008±94 |

**Claims**

1. A protein characterized in that:

a. a part of the amino acid sequence is represented by the polypeptide Met-Glu-Leu-Gln-Val-Ser-Ser-Gly-Phe-Val; and

b. which shows reactivity with an anti-$Fc_ε$ receptor antibody; and

c. which has a molecular weight of 25,000 ± 2,000; and

d. which has an isoelectric point of 5.4 to 6.0.

2. A method for producing a protein according to claim 1 which comprises culturing cells bearing a class-specific $Fc_ε$ receptor on their surface and separating and harvesting the protein from the culture supernatant.

3. A method according to claim 2, wherein said culture supernatant is treated by affinity chromatography using a carrier having bound thereto an anti $Fc_ε$ receptor anty-body and the matter adsorbed to said carrier is then separated and collected.

4. A protein according to claim 1 for use in therapy or diagnosis of allergic diseases or autoimmune diseases.

**Patentansprüche**

1. Protein, dadurch gekennzeichnet, daß

a. ein Teil der Aminosäuresequenz durch das Polypeptid Met-Glu-Leu-Gln-Val-Ser-Gly-Phe-Val repräsentiert ist, Und

b. es eine Reaktivität mit einem anti-$Fc_ε$-RezeptorAntikörper aufweist; und

c. es ein Molekulargewicht von 25000 ± 2000 hat; und

d. es einen isoelektrischen punkt von 5,4 bis 6,0 hat.

2. Verfahren zur Herstellung eines Proteins nach Anspruch 1, umfassend die Züchtung von Zellen, die auf ihrer Oberfläche einen Klassen-spezifischen $Fc_ε$-Rezeptor tragen, und die Abtrennung und das Ernten des Proteins aus dem Überstand der Kultur.

3. Verfahren nach Anspruch 2, wobei der Kulturüberstand behandelt wird durch Affinitätschromatographie mit einem Träger, an den ein anti-$Fc_ε$-Rezeptor-Antikörper gebunden ist und das an den Träger adsorbierte Material sodann abgetrennt und gesammelt wird.

4. Protein nach Anspruch 1 zur Verwendung bei der Therapie oder Diagnose von allergischen Krankheiten oder von Autoimmunkrankheiten.

## Revendications

1. Une protéine caractérisée en ce que:

a. une partie de la séquence d'acides aminés est représentée par le polypeptide
Met-Glu-Leu-Gln-Val-Ser-Gly-Phe-Val; et

b. elle présente une réactivité avec un anticorps récepteur anti-$Fc_{\varepsilon}$; et

c. elle a un poids moléculaire de 25.000 +/- 2.000

d. elle possède un point isoélectrique de 5,4 à 6,0 .

2. Une méthode de production d'une protéine selon la revendication 1 qui comprend la culture de cellules portant un récepteur $FC_{\varepsilon}$ spécifique à la classe sur leur surface et la séparation et la récolte de la protéine à partir du surnageant de la culture.

3. Une méthode selon la revendication 2 dans laquelle le dit surnageant de la culture est traité par chromatographie par affinité en utilisant un support auquel est lié un anticorps récepteur anti-$FC_{\varepsilon}$, après quoi la matière adsorbée sur le dit support est séparée et recueillie.

4. Une protéine selon la revendication 1 à utiliser dans la thérapie ou le diagnostic de maladies allergiques ou auto-immunitaires.

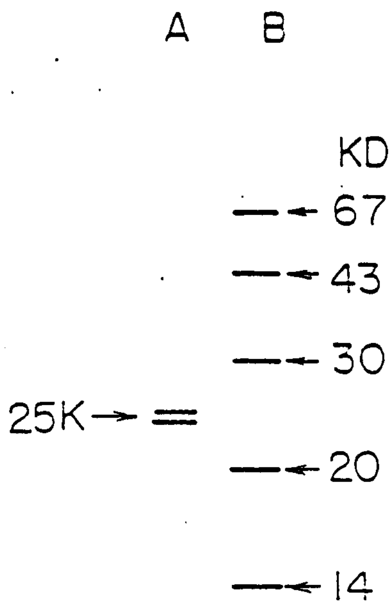


FIGURE 1

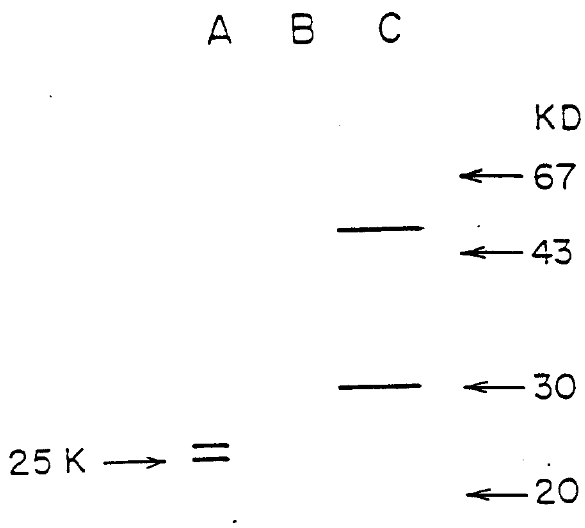


FIGURE 2

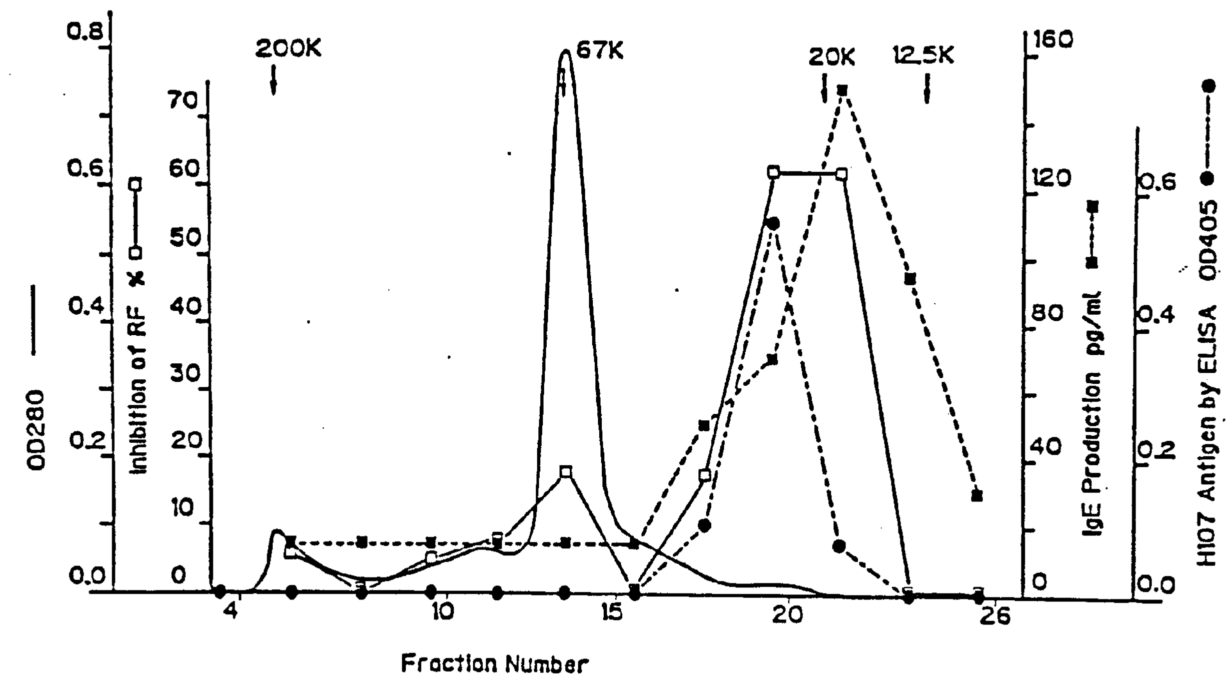

0.8
0.6
0.4
0.2
0.0
OD280
70
60
50
40
30
20
10
0
Inhibition of RF %
200K
67K
20K
12.5K
160
120
80
40
0
IgE Production pg/ml
0.6
0.4
0.2
0.0
H107 Antigen by ELISA OD405
4
10
15
20
26
Fraction Number


FIGURE 3

```
AGTGGCTCTACTTTCAGAAGAAAGTGTCTCTCTTCCTGCTTAAACCTCTGTCTCTGACGGTCCCTGCCAATCGCTCTGGTCGACCCCAACACACTAGGAGGACAGACACAGGCTCCAAAC   120

TCCACTAACCAGAGCTGTGATTGTGCCCGCTGAGTGGACTGCGTTGTCAGGGAGTGAGTGCTCCATCATCGGGAGAATCCAAGCAGGACCGCCATGGAGGAAGGTCAATATTCAGAGATC   240
                                                                                                M  E  E  G  Q  Y  S  E  I
                                                                                                1

GAGGAGCTTCCCAGGAGGCGGTGTTGCAGGCGTGGGACTCAGATCGTGCTGCTGGGGCTGGTGACCGCCGCTCTGTGGGCTGGGCTGCTGACTCTGCTTCTCCTGTGGCACTGGGACACC   360
E  E  L  P  R  R  R  C  C  R  R  G  T  Q  I  V  L  L  G  L  V  T  A  A  L  W  A  G  L  L  T  L  L  L  L  W  H  W  D  T
                           20                                                           40

ACACAGAGTCTAAAACAGCTGGAAGAGAGGGCTGCCCGGAACGTCTCTCAAGTTTCCAAGAACTTGGAAAGCCACCACGGTGACCAGATGGCGCAGAAATCCCAGTCCACGCAGATTTCA   480
T  Q  S  L  K  Q  L  E  E  R  A  A  R  N  V  S  Q  V  S  K  N  L  E  S  H  H  G  D  Q  M  A  Q  K  S  Q  S  T  Q  I  S
                           60                                                           80

CAGGAACTGGAGGAACTTCGAGCTGAACAGCAGAGATTGAAATCTCAGGACTTGGAGCTGTCCTGGAACCTGAACGGGCTTCAAGCAGATCTGAGCAGCTTCAAGTCCCAGGAATTGAAC   600
Q  E  L  E  E  L  R  A  E  Q  Q  R  L  K  S  Q  D  L  E  L  S  W  N  L  N  G  L  Q  A  D  L  S  S  F  K  S  Q  E  L  N
                           100                                                          120

GAGAGGAACGAAGCTTCAGATTTGCTGGAAAGACTCCGGGAGGAGGTGACAAAGCTAAGGATGGAGTTGCAGGTGTCCAGCGGCTTTGTGTGCAACACGTGCCCTGAAAAGTGGATCAAT   720
E  R  N  E  A  S  D  L  L  E  R  L  R  E  E  V  T  K  L  R  M  E  L  Q  V  S  S  G  F  V  C  N  T  C  P  E  K  W  I  N
                           140                                                          160

TTCCAACGGAAGTGCTACTACTTCGGCAAGGGCACCAAGCAGTGGGTCCACGCCCGGTATGCCTGTGACGACATGGAAGGGCAGCTGGTCAGCATCCACAGCCCGGAGGAGCAGGACTTC   840
F  Q  R  K  C  Y  Y  F  G  K  G  T  K  Q  W  V  H  A  R  Y  A  C  D  D  M  E  G  Q  L  V  S  I  H  S  P  E  E  Q  D  F
                           180                                                          200

CTGACCAAGCATGCCAGCCACACCGGCTCCTGGATTGGCCTTCGGAACTTGGACCTGAAGGGAGAGTTTATCTGGGTGGATGGGAGCCATGTGGACTACAGCAACTGGGCTCCAGGGGAG   960
L  T  K  H  A  S  H  T  G  S  W  I  G  L  R  N  L  D  L  K  G  E  F  I  W  V  D  G  S  H  V  D  Y  S  N  W  A  P  G  E
                           220                                                          240

CCCACCAGCCGGAGCCAGGGCGAGGACTGCGTGATGATGCGGGGCTCCGGTCGCTGGAACGACGCCTTCTGCGACCGTAAGCTGGGCGCCTGGGTGTGCGACCGGCTGGCCACATGCACG   1080
P  T  S  R  S  Q  G  E  D  C  V  M  M  R  G  S  G  R  W  N  D  A  F  C  D  R  K  L  G  A  W  V  C  D  R  L  A  T  C  T
                           260                                                          280

CCGCCAGCCAGCGAAGGTTCCGCGGAGTCCATGGGACCTGATTCAAGACCAGACCCTGACGGCCGCCTGCCCACCCCCTCTGCCCCTCTCCACTCTTGAGCATGGATACAGCCAGGCCCA   1200
P  P  A  S  E  G  S  A  E  S  M  G  P  D  S  R  P  D  P  D  G  R  L  P  T  P  S  A  P  L  H  S  *
                           300                                                          320

GAGCAAGACCCTGAAGACCCCCAACCACGGCCTAAAAGCCTCTTTGTGGCTGAAAGGTCCCTGTGACATTTTCTGCCACCCAAACGGAGGCAGCTGACACATCTCCCGCTCCTCTATGGC   1320

CCCTGCCTTCCCAGGAGTACACCCCAACAGCACCCTCTCCAGATGGGAGTGCCCCCAACAGCACCCTCTCCAGATGAGAGTACACCCCAACAGCACCCTCTCCAGATGCAGCCCCATCTC   1440

CTCAGCACCCCAGGACCTGAGTATCCCCAGCTCAGGTGGTGAGTCCTCCTGTCCAGCCTGCATCAATAAAATGGGGCAGTGATGGCCT-CCA150   1560
```

FIGURE 4

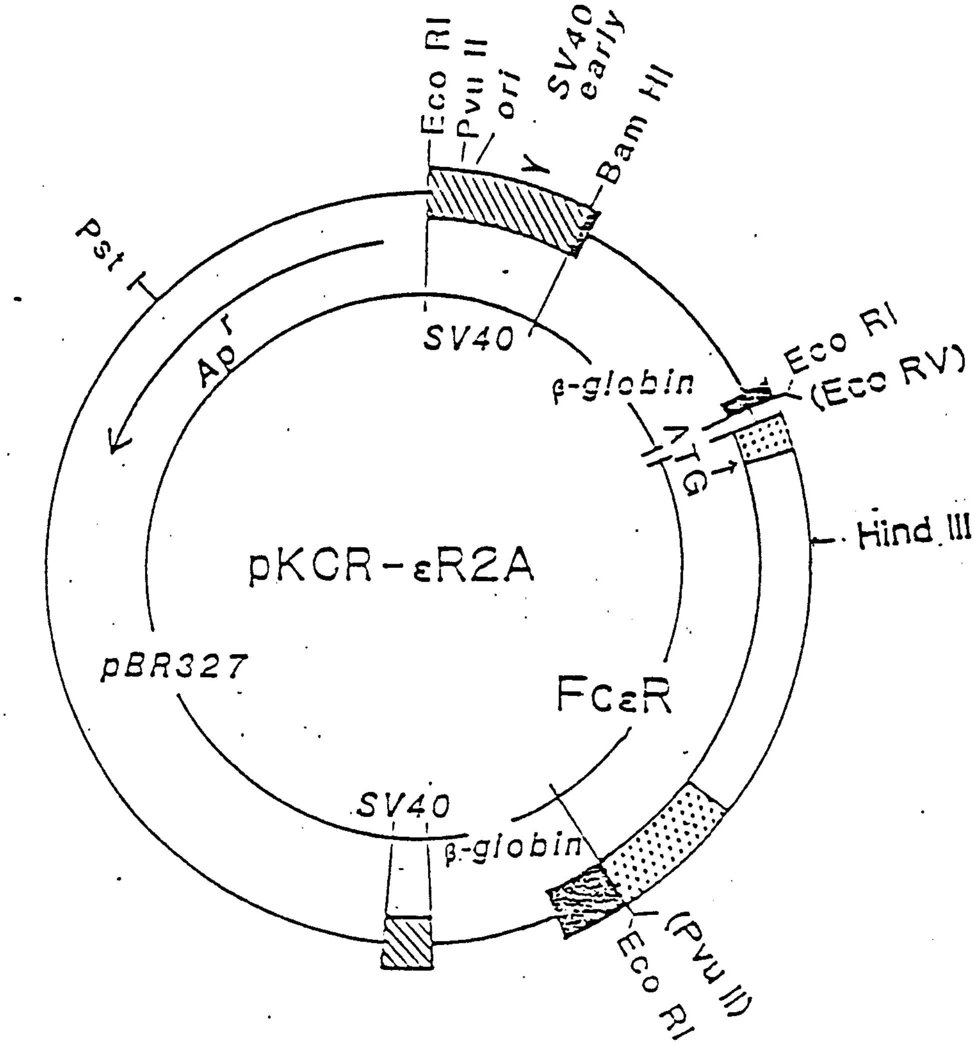
Eco RI
Pvu II
ori
SV40 early
Bam HI
Pst I
Apr
SV40
β-globin
Eco RI
(Eco RV)
ATG
Hind III
pKCR-εR2A
pBR327
FcεR
SV40
β-globin
(Pvu II)
Eco RI

FIGURE 5

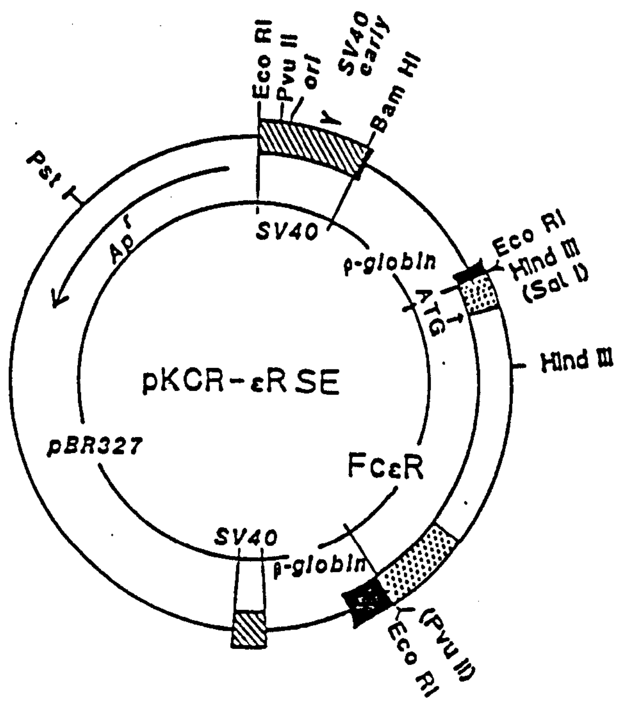
Eco RI
Pvu II
ori
SV40 early
Bam HI
Pst I
Ap^r
SV40
β-globin
Eco RI
Hind III
(Sal I)
ATG
Hind III
pKCR-εRSE
pBR327
FcεR
SV40
β-globin
(Pvu II)
Eco RI

FIGURE 6